# EUROPEAN PATENT APPLICATION

(11) **EP 1 579 865 A1**
(43) Date of publication of application: **28.09.2005**
(21) Application number: 03813729.5
(22) Date of filing: 23.06.2003
(51) Int. Cl.: A61K 35/32, A61P 5/00

(54) **MEANS FOR PREVENTING AND CURING ENDOCRINE SYSTEM DISTURBANCES**

(30) Priority: 20.12.2002 RU 2002134335
(71) Applicant: Tsygankov, Vladimir Vladimirovich, Kaluzhskaya obl., 577298 (RU)
(72) Inventor: Tsygankov, Vladimir Vladimirovich, Kaluzhskaya obl., 577298 (RU)
(74) Representative: Tollett, Ian
(86) International application number: PCT/RU2003/000272
(87) International publication number: WO 2004/056378

(57) **Abstract**

The inventive medicinal agent for preventing and treating endocrine system disturbances appears as a powder prepared from ossified reindeer horns and contains 120 IU/g of luteinizing hormone, and 54 IU/g of follicle-stimulating hormone, 2,1 IU/g of prolactin, 203 pg/g of adrenocorticotropic hormone, 2,2 ng of somatotropic hormone, thyroidal hormones including 54 fmol/g of free thyroxin, 8 fmp/g of free triiodothyronine, 4,.5 µU/g of thyreotropic hormone, and 25 pmol/g of cortisol.

## Description

### Technical Field

The present invention relates to pharmacology and medicine and has particular reference to a medicinal agent for preventing and treating endocrine system disturbances.

### Background Art

At present there are a variety of methods for correcting hormonal disturbances used in medical practice, both surgical and medicinal. As far as medicinal methods are concerned, use is as a rule made of synthetic analogs of hormones.

Known in the present state of the art are luteinizing and follicle-stimulating hormones, both relating to gonadotropic hormones which stimulate development of gonads and provide for their normal functioning. Luteinizing hormone promotes ovulation and *corpus luteum* formation, as well as secretion of androgens by the ovary. Follicle-stimulating hormone stimulates maturing of follicles in females and promotes spermatogenesis in males.

Disturbed production of said hormones gives rise to various ovarian dysfunctions, infertility, recurrent abortions, dysfunctional uterine bleeding, and so on. Used as pharmaceuticals are chorionic gonadotropin, menopausal gonadotropin and lactin.

Prolactin is a hypophysis-secreted hormone which is responsive for breast milk secretion during lactation period, dysfunction of milk secretion results in hypolactation, ovarian dysfunction, and some other endocrine disorders.

Adrenocorticotropic hormone (ACTH) is formed in the anterior pituitary lobe to stimulate secretion of adrenocortical hormones, largely glucocorticoids. Apart from that, ACTH is causative, to some extent, of splitting fats, as well as of promoting production of a pigment melanin by specific cells - melanocytes.

In clinical practice ACTH and synthetic analogs thereof are used as promoters of glucocorticoids in studying a functional state of adrenal glands and the hypothalamo-hypophyseal system, in secondary adrenocortical insufficiency, and the like.

ACTH medicinal preparation, viz, corticotropin is used with therapeutic purposes, mainly in the same instances as the preparations of corticosteroids hormones.

Somatotropic hormone (STH) is in fact a protein hormone synthesizable in the anterior pituitary lobe; principal function of said hormone is growth promotion and increase in the body size by activating anabolic processes of nitrogen metabolism. STH enhances intensity of carbohydrate, fat and mineral metabolism.

STH hyposecretion (both congenital and occurring from childhood) is causative of disturbed growth and organogenesis of internal organs. A variety of hormonal and metabolic disturbances are observed in patients as well. A hypophyseal nanism results. In case of an excess STH secretion developing most frequently in the presence of a hormone-producing pituitary tumor synthesizing STH, gigantism and acromegaly are likely to occur. To correct STH deficiency a substitution therapy exists using somatotropin injections.

Cortisol is in fact a glucocorticoid hormone influencing protein, fat and carbohydrate metabolism, as well as that of nucleic acids. Its influence on human organism is evident in a catabolic effect and increased excretion of nitrogen-bearing substances in urine. Concurrently higher glycogen deposition in the liver and increased glucose concentration in the blood stream are observed.

Disturbed synthesis of said hormone results in a disbalanced mechanism of many functions of human organism and disbolism.

Thyroid hormones (thyroxin and triiodothyronine) exert influence, first and foremost, on metabolic activity and intensity of energy metabolism, they enhance oxygen absorption by cells and tissues, promote glycogenolysis, inhibit its synthesis in the liver, and influence metabolism of fats. Effect produced by thyroid hormones on the cardiovascular system is of paramount importance. While rendering the cardiovascular system receptors more sensitive to catheholamines, thyroid hormones increases the rate of heartbeat and thereby promote arterial blood tension. Besides, thyroid hormones are indispensable for normal development and functioning of the CNS.

Thyrotropic hormone (TTH) regulates secretion of the thyroid hormones (T₃ and T₄). Disordered synthesis of said hormones is causative of a of hyper- or hypothyrosis condition. To arrest such a morbid condition use is made of synthetic analogs of thyroid hormones, as well as thyrostatics (Mashkovski M.D., Medicinal preparations. In two volumes. 11nth edition (reprint). Moscow, Meditsina Publishers, 1988, 576 pages).

All the medicinal preparations enlisted before are administered as injections. However, such a medication is rather traumatic and involves some side effects. In addition, a variety of secondary affections are likely to occur in case of overdosage or long-term administration of said preparations.

Known in the art presently is a powder-like biogenic medicinal preparation derived from ossified reindeer horns (RU, A, 2,077,887) and comprising a complex of biologically potent components of amino acids, peptides, lipids, carbohydrates, steroid hormones, fatty acids, organophosphorous compounds, iodine, as well a great many micro- and macroelements(potassium, calcium, iron, magnesium, zinc, copper, manganese, nickel, tin, chromium, lithium, baryum, and so on). It is due to a genuine composition of the powder ingredients that a diversity of effects produced on human organism is attained to provide enhanced adaptogenic resources thereof, normalize a metabolic effect including correction of lipid, protein and carbohydrate metabolism, regulation of redox and hemopoietic processes, enhancement of immunobiologic and host defenses, promotion of growth and development of osseomuscular tissue, elimination of heavy metals, toxins, etc. in humans.

The present biogenic medicinal preparation with a preserved natural bioactivity of the parent product possesses a broad spectrum of pharmacological effect which is far from having been studied comprehensively, the fact that heretofore prevents its use as a drug for correcting endocrine system disturbances.

### Disclosure of the Invention

The present invention has for its object to make use of a known medicinal preparation derived from ossified reindeer horns as an agent for prevention and treatment of endocrine system disturbances due to a general normalizing effect produced by said agent on patient's hormonal background.

Said object is accomplished due to the fact that the medicinal agent for preventing and treating endocrine system disturbances appears as a powder derived from ossified reindeer horns and comprises 120 IU/g of luteinizing hormone and 54 IU/g of follicle-stimulating hormone, 2,1 IU/ of prolactin, 203 pg/g of adrenocorticotropic hormone, 2,2 ng/g of somatotropic hormone, thyroidal hormones including 54 fmol/g of free thyroxin, 8 fmp/g of free triiodothyronine, 4,5 µU/g of thyreotropic hormone, and 25 pmol/g of cortisol.

The content of prolactin, adrenocorticotropic hormone, somatotropic hormone and cortisol is detected using isotope methods, while that of luteinizing hormone, follicle-stimulating hormone, thyreotropic hormone, free thyroxin and free triiodothyronine is found by immunoenzyme methods (that is, enhanced luminescence and delayed fluorescence).

Hence the herein-proposed composition comprises the component that make up a definitely balanced system owing to which the composition is in possession of such a versatile activity, whereby the composition can find application in prevention and treatment of endocrine system disturbances.

### Best Method of Carrying out the Invention

The herein-proposed medicinal preparation appearing as a powder derived from ossified reindeer horns comprises 120 IU/g of luteinizing hormone and 54 IU/g of follicle-stimulating hormone. Said content of the hormones provides for a desired therapeutic effect in a combination treatment of the aforementioned morbid conditions. Prolactin in an amount of 2,1 IU/g produces a correcting effect in hypophyseal insufficiency, causing no negative action on healthy human organism. Adrenocorticotropic hormone contained in the preparation in amount of 203 pg/g, when applied in a complex therapy, makes it possible correct a general hormonal background of human organism. The presence of somatotropic hormone (2,2 ng/g) in the preparation makes possible correcting, to some extent or other, the available hormonal disturbances resulting from a deficiency of said hormone, as well as provide help to the organism in the periods of active growth in children and adolescents.

The preparation also contains 25 pmole/g of cortisol, whereby the drug can be used for correcting morbid conditions concerned with disordered synthesis of said hormone.

The preparation comprises also thyroid hormones, i.e., free thyroxin (54 fmol/g) and free triiodothyronine (8 fmp/g), as well as thyreotropic hormone (4,5 µU/g). Such an amount and a harmonious combination of thyroid hormones produce a normalizing effect on the thyroid gland functions and on a hormonal status as a whole.

The proposed medicinal agent is prepared by disintegrating ossified reindeer horns to obtain porous particles of the disintegrated product. The fineness of the product particles is principally below 0,25 mm, a specific surface area being about 100 m²/g and a volume of pores of about 3,0 m³/g.

The herein-proposed medicinal agent based on ossified reindeer horns is a complex drug capable of normalizing metabolism, restoring disturbed functions of endocrine glands and normalizing hormonal status. The aforementioned pituitary and thyroid hormones are in such microdoses that are absolutely harmless for healthy humans but have a positive correcting effect in case of a disturbed or disbalanced endocrine status of human organism. An extra-stabilizing affect is attained also due to a combination of said hormones with micro- and macroelements, thus providing a balanced assimilation of said hormones.

An optimum dosage, which enables one to mildly and progressively correct current hormonal disturbances (both by a single-drug or complex therapy), is 0,4 g two or three times a day for 1-2 months.

The preparation is distinguished for mild and efficient effect produced freedom from any side effects and simple application.

Efficient use of the proposed agent is corroborated by the examples adduced below.

### 1. Exemplary treatment of morbid conditions of endocrine system.

Female patient, 30, the diagnosis being one of secondary infertility; the patient has been failing to become pregnant for a ten-year period. After three courses of administration of the proposed agent appearing as a powder derived from ossified reindeer horns and comprising 120 IU/g of luteinizing hormone and 54 IU/g of follicle-stimulating hormone, 2,1 IU/g of prolactin, 203 pg/g of adrenocorticotropic hormone, 2,2 ng/g of somatotropic hormone, thyroidal hormones including 54 fmol/g of free thyroxin, 8 fmp/g of free triiodothyronine, 4,5 µU/g of thyreotropic hormone, and 25 pmol/g of cortisol in a dosage of 0,8 g twice a day, pregnancy set in, terminated in a successful childbirth.

### 2. Exemplary preventive treatment of endocrine system disturbances.

Female patient, 50, having residence in an endemic area (with low water iodine content) have been for a long time suffering with degree III diffuse endemic goiter, the patient being in euthyroid state. Upon one-year administration of the proposed medicinal agent appearing as a powder derived from ossified reindeer horns and comprising 120 IU/g of luteinizing hormone, and 54 IU/g of follicle-stimulating hormone, 2,1 IU/g of prolactin, 203 pg/g of adrenocorticotropic hormone, 2,2 ng/g of somatotropic hormone, thyroidal hormones including 54 fmol/g of free thyroxin, 8 fmp/g of free triiodothyronine, 4,5 µU/g of thyreotropic hormone, and 25 pmol/g of cortisol in a dosage of 0,8 g twice a day in courses with a dosage of 0,4 g twice a day, the goiter reduces down to degree I, the level of hormones remaining normal. The patient continues receiving the drug in successive courses, her status presence being satisfactory. No complaints on the patient's part.

Female patient, 30, has been suffering from dysmenorrhea since the age of 14; the patient has for a long time been taking the proposed medicinal agent appearing as a powder derived from ossified reindeer horns and comprising 120 IU/g of luteinizing hormone, and 54 IU/g of follicle-stimulating hormone, 2,1 IU/g of prolactin, 203 pg/g of adrenocorticotropic hormone, 2,2 ng/g of somatotropic hormone, thyroidal hormones including 54 fmol/g of free thyroxin, 8 fmp/g of free triiodothyronine, 4.5 µU/g of thyreotropic hormone, and 25 pmol/g of cortisol in a dosage with a dosage of 0,4 g twice a day, in successive courses; normalized menstrual cycle in the patient results.

### Industrial Applicability

The present invention can find application to good advantage for preventing and treating endocrine system disturbances due to a general normalizing effect produced by said agent on patient's hormonal background.

## Claims

1. A medicinal agent for preventing and treating endocrine system disturbances, **CHARACTERIZED in that** it appears as a powder prepared from ossified reindeer horns and contains 120 IU/g of luteinizing hormone, and 54 IU/g of follicle-stimulating hormone, 2,1 IU/g of prolactin, 203 pg/g of adrenocorticotropic hormone, 2,2 ng/g of somatotropic hormone, thyroidal hormones including 54 fmol/g of free thyroxin, 8 fmp/g of free triiodothyronine, 4,5 µU/g of thyreotropic hormone, and 25 pmol/g of cortisol.
